(19) Europäisches Patentamt
European Patent Office
Office européen des brevets

(11) **EP 1 020 532 B1**

(12) **EUROPEAN PATENT SPECIFICATION**

(45) Date of publication and mention
of the grant of the patent:
**08.04.2009 Bulletin 2009/15**

(51) Int Cl.:
*C12Q 1/60* [(2006.01)]     *G01N 33/536* [(2006.01)]
*G01N 33/92* [(2006.01)]     *G01N 33/52* [(2006.01)]

(21) Application number: **98928635.6**

(22) Date of filing: **22.06.1998**

(86) International application number:
**PCT/JP1998/002795**

(87) International publication number:
**WO 1998/059068 (30.12.1998 Gazette 1998/52)**

(54) **METHOD FOR ASSAYING BIOLOGICAL SPECIMENS FOR SUBSTANCES CONTAINED IN THE COMPONENTS THEREOF AND REAGENT TO BE USED IN THIS METHOD**

METHODE UND REAGENZIEN ZUM BESTIMMEN VON SUBSTANZEN IN BIOLOGISCHEN PROBEN

PROCEDE SERVANT A ANALYSER DES SPECIMENS BIOLOGIQUES AFIN DE RECHERCHER DES SUBSTANCES CONTENUES DANS LEURS CONSTITUANTS ET REACTIF UTILISE DANS CE PROCEDE

(84) Designated Contracting States:
**DE ES FR GB**

(30) Priority: **25.06.1997 JP 16928197**

(43) Date of publication of application:
**19.07.2000 Bulletin 2000/29**

(73) Proprietor: **SYSMEX CORPORATION**
**Kobe-shi,**
**Hyogo 651-0073 (JP)**

(72) Inventors:
• **KISHI, Koji,**
**Int. Reagents Co.,**
**Res. & Dev. Cent.**
**Kobe-shi,**
**Hyogo 651-2241 (JP)**
• **KAKUYAMA, Tsutomu,**
**Int. Reagents Co.**
**Nishi-ku,**
**Kobe-shi,**
**Hyogo 651-2241 (JP)**
• **SHIRAHASE, Yasushi,**
**Int. Reagents Co.**
**Nishi-ku,**
**Kobe-shi,**
**Hyogo 651-2241 (JP)**
• **WATAZU, Yoshifumi,**
**Int. Reagents Co.**
**Nishi-ku,**
**Kobe-shi,**
**Hyogo 651-2241 (JP)**

(74) Representative: **Rapp, Bertram et al**
**Charrier Rapp & Liebau,**
**Postfach 31 02 60**
**86063 Augsburg (DE)**

(56) References cited:
WO-A1-94/04483          JP-A- 01 503 596
JP-A- 06 242 110         JP-A- 08 131 197
JP-A- 08 503 937         US-A- 5 409 959

• CHEMICAL ABSTRACTS, vol. 131, no. 4, 26 July 1999 (1999-07-26) Columbus, Ohio, US; abstract no. 41640, XP002144468 & K. KISHI ET AL.: "New homogeneous assay method for serum LDL-cholesterol by using cholesterol dehydrogenase" SEIBUTSU SHIRYO BUNSEKI , vol. 21, no. 5, 1 May 1998 (1998-05-01), pages 385-392, Japan
• CHEMICAL ABSTRACTS, vol. 126, no. 6, 10 February 1997 (1997-02-10) Columbus, Ohio, US; abstract no. 83819, XP002144469 & J. HARTMANN ET AL.: " Supramolecular interactions on mass sensitive sensors in gas phases and liquids" SENSORS AND ACTUATORS , vol. B34, no. 1-3, 1996, pages 305-311, Amsterdam NL

- **IKEDA M. ET AL: 'New homogeneous assay for serum LDL-cholesterol by using cholesterol dehydrogenase' CLINICAL CHEMISTRY vol. 44, no. 6(2), 01 June 1998, 50TH ANNUAL MEETING OF THE AMERICAN ASSOCIATION OF CLINICAL CHEMISTRY; CHICAGO, ILLINOIS, USA; AUGUST 2-6, 1998, page A81**

- **KAKUYAMA T. ET AL: 'New homogeneous assays for estimation of serum HDL and LDL-cholesterol levels' CLINICAL CHEMISTRY vol. 47, no. S6, 01 June 2001, 53RD ANNUAL MEETING OF THE AACC/CSCC; CHICAGO, ILLINOIS, USA; JULY 29-AUGUST 02, 2001, pages A55 - A56**

**Description**

TECHNICAL FIELD

The present invention relates to a method for assaying cholesterol in a low density lipoprotein or in a high density lipoprotein in biological specimens in the field of clinical diagnosis and to a reagent for use therein. More specifically, the present invention relates to a method for assaying high density lipoprotein (HDL) for cholesterol and to a reagent for use therein.

BACKGROUND ART

Lipoproteins have from since been fractionated by ultracentrifugation operation into high density lipoprotein (HDL, density: 1.063-1.21), low density lipoprotein (LDL, density: 1.019-1.063), very low density lipoprotein (VLDL, density: 1.006-1.019), and chylomicron (CM, density<1.006). The fractionation operation needs an ultracentrifuge and in addition must continue centrifugation for several days so that it is impossible to treat many samples.

In place of this, a method has become the main current in which by mixing serum with a solution which comprises a precipitation agent that contains both a polyethylene glycol or a polyanion such as dextransulfate and a divalent cation such as calcium, or both phosphorous tungstic acid and a divalent cation, LDL, VLDL, and CM are precipitated and only HDL that remains in the supernatant after the centrifugation is fractionated. In this method, use can be made of an automatic analyzing apparatus which has widely prevailed in the field of clinical assays. That is, since the assay of total cholesterol using an enzyme assay method has been established by use of an automatic analyzing apparatus, utilization of the established assay method has enabled the measurement of concentration of cholesterol in the fractionated HDL.

However, this method still needs centrifugation operation though at a low speed and there has been the problem of artificial errors in quantitative determination or misplacement of samples when the precipitation agent and serum are mixed with each other. In addition, it has been impossible to measure other general biochemical items simultaneously by use of an automatic analyzing apparatus. Clinical assays are demanded to be carried out speedily, which also give rise to the desire for a shortening of test time.

On the other hand, from clinical point of view, there has been a report which lays importance on the level of cholesterol in LDL, which is a risk factor for arteriosclerosis ("Standard Level for Total Cholesterol and Basis for Its Setting", Domyaku Koka (Arteriosclerosis), 24 (6), 260 (1966)). Currently, the level of cholesterol in LDL is obtained from measured values of total cholesterol (T-CHO), neutral fats (TG) and cholesterol in HDL according to an equation by introducing an empirical factor therein. The equation (Friedewald WT, et al., Clin. Chem., 18, 499-502 (1972)) is as shown below;

$$\text{Cholesterol value in LDL} = (\text{T-CHO value}) - (\text{Cholesterol value in HDL}) - (\text{TG value}) /5.$$

In this method, all the three terms to be measured must be exactly measured before the equation can be valid. It is said that the calculated value does not reflect the concentration of cholesterol in LDL when TG value exceeds 400 mg/dl or the concentration of cholesterol in LDL is 100 mg/dl or less (Warnick GR, et al., Clin. Chem., 36(1), 15-19 (1990), McNamara, JR, et al., Clin. Chem., 36(1), 36-42 (1990)). Therefore, extraordinary values which are the target of assay cannot be attained by this method. Besides, there have been a method in which lipoprotein is isolated by electrophoresis and the quantity of protein is measured or a method in which cholesterol is measured for respective lipoproteins by HPLC. However, both of them are deficient in the ability to treat many samples and need an expensive apparatus.

Recently, in order to dissolve the above-described problems associated with the measurement of cholesterol in HDL, an automatic kit for the measurement of cholesterol in HDL has been developed and prevailing. However, the technologies described in the Japanese Registered Patent No. 2600065, Japanese Patent Application Laid-open No. Hei 8-201393 (published as JP-A-8201393) and Japanese Patent Application Laid-open No. Hei 8-31195 (published as JP-A-8031195) use a precipitation agent in combination and the metal used as a divalent cation contained in the precipitation agent forms insoluble precipitation by the action of a detergent generally used in an automatic analyzing apparatus, and the precipitation accumulates in a waste disposal mechanism, which causes a disorder of the automatic analyzing apparatus. Further, insoluble agglutinates are formed in the reaction mixture and the agglutinates not only make the reaction mixture turbid, which could affect the results of measurement, and cause an error in measurement, but also stain the reaction cell to give not a small influence on the results of measurement of other biochemical items simultaneously measured.

In most automatic analyzing apparatus now prevailing, the reaction is often completed in 10 minutes. In this case, the occurrence of a change in turbidity, if any, casts a question on the accuracy of the measured values. In addition, the problem also arises that the turbidity of reaction mixture deteriorates reproducibility. Therefore, samples which can be measured in the apparatus are limited and a wide range of measuring wavelengths and various types of samples from patients cannot be served. For example, it has the defect that in the vicinity of 340 nm (UV region), the absorbance is 2 or more, or 3 or more and frequently exceeds the tolerance limits of the analyzing apparatus, due to the turbidity caused by the agglutinates.

The technology described in Japanese Patent Application Laid-open No. Hei 9-96637 (published as JP-A-9096637), which uses a divalent cation, is a method involving use of an antiserum contained in the reaction mixture which forms agglutinates with a lipoprotein. This also forms insoluble antigen-antibody agglutinates so that the reaction cell is stained. Therefore, no small an influence is given on the results of measurement of other biochemical items that are measured simultaneously. Furthermore, the high turbidity in the reaction mixture makes accurate measurement impossible for the cholesterol in HDL in particular in UV region because of the same causes as described above. In a longer wavelength region, the measured values are inaccurate due to the influence of turbidity.

Japanese Patent Application Laid-open No. Hei 6-242110 (published as JP-A-6242110) discloses a method using a technology intended to finally eliminate such turbidity. However, this method cannot be applied in many cases to an automatic analyzing apparatus since the technology needs at least 3 to 4 steps of operations for dispensing a reagent and generally prevailing automatic analyzing apparatuses for biochemical items are in the main those which involve at most 2 steps of such operations.

JP-A-8 131 197 relates to an assay for the determination of cholesterol in high density lipoproteins (HDL) by aggregating lipoproteins other than HDL with e.g. cyclodextrines.

On the other hand, in the case of measurement of cholesterol in LDL, it is a current status that a calculation method has to be taken.

An object of the present invention is to provide a method for assaying cholesterol in HDL or LDL in biological specimens by use of a general-purpose automatic analyzing apparatus, without separation of the specimen by centrifugation operation and to provide a reagent for use therein.

DISCLOSURE OF THE INVENTION

The present inventors have made intensive study with view to solving the above-described problems and as a result they have found that calixarenes, which have been developed for inclusion of metal, etc. (Rogers, R.D., et al., J. Radioanal. Nucl. Chem., 208, 153-161 (1996)), are useful for fractional quantitative determination of the components in, for example, some kinds of lipoprotein without separation operations.

That is, the present invention relates to a reagent as defined in claims 4 to 8 containing calixarenes for assaying cholesterol in LDL or HDL in biological specimens, the use of said reagents for assaying cholesterol in LDL or HDL as defined in present claims 9 and 10 and to a method as defined in claims 1 to 3 for assaying cholesterol in LDL or HDL in biological specimens by use of calixarenes, wherein one or more of the calixarenes form a complex with a component or components in a biological specimen.

The components containing cholesterol in a biological specimen mean specified components contained in serum or plasma, for example, HDL, and LDL. The substances in the components in each specimen mean substances contained in the components in the biological specimens that are the target of analysis, for example, cholesterol, when the component in the biological specimens are lipoproteins.

Calixarenes, which are constituted of the unit of phenol, comprise a cyclic oligomer of 4 to 8 phenol molecules bonded one to another through a methylene group to form a ring. Calixarenes include calix(4)arene, calix(6)arene, calix(8)arene, calix(4)arene sulfate, calix(6)arene sulfate, calix(8)arene sulfate, calix(4)arene acetate, calix(6)arene acetate, calix(8)arene acetate, carboxycalix(4)arene, carboxycalix(6)arene, carboxycalix(8)arene, calix(4)areneamine, calix(6)areneamine, calix(8)areneamine, etc. In the present invention, one or more kinds selected from these calixarenes can be used and upon use no limitation is posed. Calixarene sulfate, which has been successfully commercialized, is excellent in water solubility so that it is easy to handle. When the calixarenes are applied to the measurement of cholesterol, they may be added in the reaction mixture in an amount of 0.05 to 20 mmol/l, preferably 0.1 to 5 mmol/l, under the conditions where cholesterol can be measured enzymatically.

The assay in the present invention means measurement of the substances in the components in biological specimens, and to determine them by quality or by quantity.

That calixarenes form complexes with components in a biological specimen means that calixarenes bind with components in a biological specimen and the manner of binding is not limited particularly so far as the calixarenes bind with the components in the biological specimen and it includes, for example, ionic bonding, coordination bonding, etc.

Lipoproteins include a lipid inside and a protein outside as a result of binding of a lipid component containing cholesterol with an apoprotein and are solubilized in a living organism. Calixarenes form complexes mainly with LDL through ionic bonding on the surface of the lipoprotein to inhibit the release of the components (for example, cholesterol and neutral fats) in the lipoproteins. Therefore, the formation of a complex means the inhibition of an enzyme reaction of a substance contained in the components in the biological specimens in which the complex is formed and this enables the measurement of the target substance in the components in the biological specimens by an enzyme reaction. For example, the cholesterol or neutral fats in HDL can be determined by inhibiting the enzyme reaction of cholesterol or neutral fats in LDL.

The pH conditions of reaction mixture depend on the substance to be assayed and when the cholesterol in HDL, for example is to be measured, may be those conditions under which the measurement of total cholesterol is completed, usually in the range of pH 6.0 to 9.0.

In the present invention, the component in biological specimens as a target of assay can be selected by changing the concentration of calixarenes. For example, LDL is selected when the concentration is below 0.7 mM, and HDL is selected when the concentration is in the range of 0.7 to 5 mM, and the substances in the components in biological specimens can be measured. The concentration ranges indicated here are not unique since optimal concentrations vary depending on the conditions of measurement, such as pH, and are determined by experiment under respective measurement conditions.

Thus, that the components in biological specimens containing substances as a target for measurement can be selected by changing the concentrations of calixarenes is useful in their application in the field of extracorporeal diagnostic medicaments. For example, in the conventional methods, the measurement of cholesterol in HDL was carried out by use of a method limited to the measurement of only the substance that is the target of measurement (cholesterol) and the method cannot be applied to the measurement of substances other than the cholesterol in the same HDL, for example, the neutral fats in HDL. In the measurement of the same cholesterol, the method is effective only for the cholesterol in HDL and is not applicable to other lipoproteins, for example, the cholesterol in LDL. The present invention is characterized in that it can be applied to the assay of different substances contained in the same component in the biological specimen. Therefore, the cholesterol contained in HDL, or LDL can be made to be the targets of measurement.

The reagent of the present invention is provided usually as a reagent kit in which calixarenes are blended with a known reagent for measurement suitable for the measurement of substances in the components in biological specimens such that the final concentrations of calixarenes are desired concentrations, or as a reagent kit in which a reagent containing calixarenes adjusted to desired concentrations is combined with a known reagent for measurement. The form of reagent provided may be in a dry state or in a liquid state and is not limited particularly. The reagent kit may be blended with activators for enzymes. The reagent kit may contain a combination of different kinds of reagents which differ in the timing of addition in the reaction mixture, for example, a combination of a reagent for a first reaction and a reagent for a second reaction.

The method of assay according to the present invention includes a mode in which calixarenes are added such that their final concentrations are desired concentrations upon preparation by adding a buffer solution to various commercially available reagents for measurement.

The reagent of the present invention is prepared appropriately so as to contain calixarenes and reagents such as enzymes which react with the target substances in the components in biological specimens as the other components. For example, the level of cholesterol in HDL or LDL can be measured by the method for measuring total cholesterol. In this case, use is made of one or more kinds of enzymes selected from the group consisting of lipoprotein lipase (LIP), cholesterol esterase (CE), cholesterol dehydrogenase (CDH, for example, nicotinamide adenine dinucleotide dependent cholesterol dehydrogenase, nicotinamide adenine dinucleotide phosphate dependent cholesterol dehydrogenase, etc.) and cholesterol oxidase (CO). Furthermore, appropriate selection is made of activators, stabilizers, coenzymes, peroxidase (PO) for the measurement of hydrogen peroxide, and colorants which are added under the conditions for completing the reaction for the measurement of cholesterol.

When CDH is used, a technology of adding hydrazines for the purpose of inhibiting a reverse reaction of the enzyme has previously been reported (Japanese Patent Application Laid-open No. Hei 5-176797). Also, in the present invention, assays can be carried out under the conditions under which hydrazines are added. There is the technology of chemically modifying enzymes so that the enzymes can have selectivity (Japanese Registered Patent No. 2600065). In the present invention, such enzymes can be used. However, in the present invention, sufficient selectivity can be obtained without using such enzymes.

Coenzymes used in the case where CDH is used include β-nicotinamide adenine dinucleotide, oxidation form (NAD), Thio-nicotinamide adenine dinucleotide, oxidation form (t-NAD), β-nicotinamide adenine dinucleotide phosphate, oxidation form (NADP), Thio-nicotinamide adenine dinucleotide phosphate, oxidation form (t-NADP), etc. and one or more coenzymes selected from the group consisting of these are used. In the presence of cholesterol, these coenzymes are converted into respective reduction forms, i.e., NADH, t-NADH, NADPH, and t-NADPH.

The cholates and detergents used as an activator for enzymes in the measurement of total cholesterol may be selected appropriately under the conditions under which cholesterol is measured with enzymes and used after adjusting the concentration by experiment. For example, in the case where the cholesterol in HDL is to be measured, in a first reaction, calixarenes may be added to lipoproteins other than HDL to form a complex for stabilization, and in a second reaction, an enzyme, etc. may be added to measure the concentration of the cholesterol in HDL.

In the case where the cholesterol in LDL is to be measured, a combination of the conditions previously described is used and a complex with LDL is formed for stabilization in a first reaction while the cholesterol in HDL is eliminated by preliminary reaction and the remaining cholesterol in LDL is measured in a second reaction.

The combinations and concentrations of calixarenes which form complexes with lipoproteins in the first reaction may be adjusted. In the measurement of cholesterol, enzyme reactions using 3 types of enzyme (1) CDH, (2) COD and LIP, and (3) CE in combination may be used. In the second reaction, detergents, cholates, enzymes, etc. for decomposing the LDL complex are added and measurement is made of the cholesterol in LDL which is not reacted in the first reaction, then the concentration of the cholesterol in LDL can be obtained by a deduction from the concentration of cholesterol in the first reaction. The compounds added in order to decompose LDL are not limited particularly so long as they do not interfere with the enzymatic measurement of cholesterol.

Hereafter, the present invention will be described in detail by examples of measurement of cholesterols in HDL and LDL.

[Example 1]

The following reagents 1 and 2 were prepared and HDL-cholesterol was measured.

| Preparation of Reagent 1 | |
| --- | --- |
| Buffer solution | pH 6.5 |
| Calix(6)arene sulfate | 1.0 mmol/l |
| Hydrazinium dichloride | 100 mmol/l |
| β-nicotinamide adenine dinucleotide, oxidation form (NAD) | 5.0 mmol/l |

| Preparation of Reagent 2 | |
| --- | --- |
| Buffer solution | pH 8.5 |
| Cholesterol dehydrogenase | 20.0 U/ml |
| Cholesterol esterase | 6.0 U/ml |

As samples were used 25 serum samples from ordinary persons. Measurement was practiced using an automatic analyzing apparatus (Hitachi 7170 Type Automatic Analyzing Apparatus). The operation was made by first adding 180 μl of Reagent 1 to 5 μl of each sample and incubating each mixture at 37°C for 5 minutes and at this point in time Absorbance 1 was measured at a main wavelength of 340 nm and a sub wavelength of 570 nm.

Further, 60 μl of Reagent 2 was added to each sample and the resulting mixtures were incubated at 37°C for 5 minutes and at this point in time Absorbance 2 was measured at a main wavelength of 340 nm and at a sub wavelength of 570 nm. A difference between Absorbance 1 and Absorbance 2 was obtained and the value of sample was calculated based on the control of a known HDL-cholesterol concentration as a standard solution. As a control method, the polyethylene glycol (PG) method was used. The PG method was carried out using PG POL manufactured by International Reagents Corporation. The concentration of cholesterol in the supernatant after centrifugation was obtained by use of T-CHC Reagent A manufactured by International Reagents Corporation. The results obtained are shown in Table 1.

Correlation with the control method was as follows: correlation coefficient: 0.993, regression equation: $Y = 0.989X + 1.18$, thus giving a good correlation. Note that Y in the regression equation is the value based on Example 1 (unit: mg/dl) and X is the value obtained by the PG method (unit: mg/dl).

Table 1 Results of Measurements (Unit: mg/dl)

| Sample No. | Method of Invention | Control Method |
| --- | --- | --- |
| 1 | 39.2 | 42.2 |
| 2 | 45.5 | 43.2 |
| 3 | 57.7 | 56.4 |
| 4 | 47.2 | 46.2 |
| 5 | 75.4 | 74.4 |
| 6 | 41.9 | 39.9 |

(continued)

| Sample No. | Method of Invention | Control Method |
|---|---|---|
| 7 | 37.4 | 36.6 |
| 8 | 94.3 | 88.5 |
| 9 | 50.1 | 49.3 |
| 10 | 85.9 | 91.7 |
| 11 | 95.1 | 92.6 |
| 12 | 39.1 | 39.5 |
| 13 | 29.7 | 28.0 |
| 14 | 61.8 | 62.1 |
| 15 | 52.5 | 51.0 |
| 16 | 63.6 | 61.9 |
| 17 | 69.6 | 71.0 |
| 18 | 81.4 | 79.9 |
| 19 | 27.0 | 25.0 |
| 20 | 50.0 | 50.5 |
| 21 | 40.4 | 39.5 |
| 22 | 74.9 | 77.0 |
| 23 | 69.2 | 67.2 |
| 24 | 52.8 | 54.1 |
| 25 | 68.8 | 68.6 |

[Example 2]

The following reagents 1 and 2 were prepared and HDL-cholesterol was measured.

| | |
|---|---|
| Preparation of Reagent 1 | |
| Buffer solution | pH 6.5 |
| Sodium N-(2-hydroxy-3-sulfopropyl)-3,5-dimethoxyaniline | 1.0 mmol/l |
| Calix (6) arene sulfate | 1.0 mmol/l |
| Peroxidase | 5.0 U/l |
| | |
| Preparation of Reagent 2 | |
| Buffer solution | pH 7.0 |
| Cholesterol oxidase | 10.0 U/ml |
| Cholesterol esterase | 6.0 U/ml |
| 4-Aminoantipyrine | 10.0 mmol/l |

As samples were used 20 serum samples from ordinary persons. Measurement was practiced using an automatic analyzing apparatus (Hitachi 7170 Type Automatic Analyzing Apparatus). The operation was made by first adding 180 μl of Reagent 1 to 3 μl of each sample and incubating each mixture at 37°C for 5 minutes and at this point in time Absorbance 1 was measured at a main wavelength of 600 nm and a sub wavelength of 700 nm.

Further, 60 μl of Reagent 2 was added to each sample and the resulting mixtures were incubated at 37°C for 5 minutes and at this point in time Absorbance 2 was measured at a main wavelength of 600 nm and at a sub wavelength of 700 nm. A difference between Absorbance 1 and Absorbance 2 was obtained and the value of sample was calculated based on the control of a known HDL-cholesterol concentration as a standard solution. As a control method, the polyethylene glycol (PG) method was used. The PG method was carried out in the same manner as in Example 1. The results obtained are shown in Table 2.

Correlation with the control method was as follows: correlation coefficient: 0.986, regression equation: $Y = 0.879X + 6.03$, thus giving a good correlation. Note that Y in the regression equation is the value based on Example 2 (unit: mg/dl) and X is the value obtained by the PG method (unit: mg/dl).

Table 2 Results of Measurements (Unit: mg/dl)

| Sample No. | Method of Invention | Control Method |
|---|---|---|
| 1 | 49.2 | 50.9 |
| 2 | 43.9 | 45.4 |
| 3 | 51.9 | 58.0 |
| 4 | 51.0 | 51.9 |
| 5 | 66.9 | 71.4 |
| 6 | 70.4 | 71.1 |
| 7 | 47.6 | 43.3 |
| 8 | 28.1 | 25.0 |
| 9 | 34.8 | 32.4 |
| 10 | 69.7 | 73.3 |
| 11 | 41.7 | 41.5 |
| 12 | 60.8 | 62.0 |
| 13 | 102.1 | 104.3 |
| 14 | 42.5 | 47.2 |
| 15 | 47.1 | 45.9 |
| 16 | 63.6 | 67.3 |
| 17 | 45.6 | 42.8 |
| 18 | 42.3 | 37.6 |
| 19 | 52.5 | 54.9 |
| 20 | 37.0 | 30.1 |

[Example 3]

The following reagents 1 and 2 were prepared and LDL-cholesterol was measured.

Preparation of Reagent 1
Buffer solution      pH 7.5
Calix(6)arene sulfate      0.8 mmol/l
Hydrazinium dichloride      100 mmol/l
β-nicotinamide adenine dinucleotide, oxidation form (NAD)      5.0 mmol/l
Cholesterol dehydrogenase      10.0 U/ml
Cholesterol esterase      4.0 U/ml

Preparation of Reagent 2
Buffer solution      pH 7.5
Cholesterol esterase      3.0 U/ml

As samples were used 24 serum samples from ordinary persons and values of samples were calculated using as a standard solution the control of a known LDL-cholesterol concentration whose conversion is known in accordance with the Friedewald formula. Measurement was practiced using an automatic analyzing apparatus (Hitachi 7170 Type Automatic Analyzing Apparatus). The operation was made by first adding 210 μl of Reagent 1 to 3 μl of each sample and incubating each mixture at 37°C for 5 minutes and at this point in time Absorbance 1 was measured at a main wavelength of 340 nm and a sub wavelength of 570 nm.

Further, 70 μl of Reagent 2 was added to each sample and the resulting mixtures were incubated at 37°C for 5 minutes and at this point in time Absorbance 2 was measured at a main wavelength of 340 nm and at a sub wavelength of 570 nm. A difference between Absorbance 1 and Absorbance 2 was obtained and the value of sample (unit: mg/dl) was calculated based on the control of a known LDL-cholesterol concentration as the standard solution.

As a control method, use is made of T-CHO Reagent KL "KOKUSAI" and TG Reagent A, both manufactured by International Reagents Corporation, and PG POL method and the concentration of LDL-cholesterol was obtained in accordance with the Friedewald formula. The results obtained are shown in Table 3.

Correlation with the control method was as follows: correlation coefficient: 0.982, regression equation: Y = 1.008X + 5.98, thus giving a good correlation. Note that Y in the regression equation is the value based on Example 3 (unit: mg/dl) and X is the value obtained by the control method (unit: mg/dl).

Table 3 Results of Measurements (Unit: mg/dl)

| Sample No. | Method of Invention | Control Method |
|---|---|---|
| 1 | 49.8 | 60.8 |
| 2 | 121.0 | 120.7 |
| 3 | 144.8 | 144.8 |
| 4 | 85.9 | 88.9 |
| 5 | 70.3 | 60.8 |
| 6 | 93.1 | 81.1 |
| 7 | 105.5 | 92.1 |
| 8 | 116.0 | 103.1 |
| 9 | 130.0 | 108.2 |
| 10 | 129.7 | 116.2 |
| 11 | 139.2 | 126.5 |
| 12 | 154.3 | 133.7 |
| 13 | 160.9 | 151.3 |
| 14 | 189.7 | 173.0 |
| 15 | 243.5 | 236.9 |
| 16 | 77.5 | 79.3 |
| 17 | 165.5 | 173.9 |
| 18 | 168.3 | 170.3 |
| 19 | 121.6 | 118.2 |
| 20 | 93.6 | 87.3 |
| 21 | 99.0 | 92.7 |
| 22 | 70.7 | 63.8 |
| 23 | 175.7 | 164.0 |
| 24 | 112.2 | 99.3 |

INDUSTRIAL APPLICABILITY

According to the present invention, substances in the components in biological specimens, for example, cholesterol in a specified fraction in serum (lipoprotein, etc.) can be assayed without separation from any other fractions. Therefore, since separation by fractionation such as centrifugation fractionation is unnecessary, the method of the present invention is easy in operation so that errors in measurement and artificially caused problems can be decreased.
Furthermore, the method of the present invention can be applied to methods using two kinds of reagents so that continuous measurement using a general-purpose automatic analyzing apparatus is possible, which enables multichannel measurement in combination with other test items.
The present application is based on Japanese Patent Application No. Hei 9-169281 filed in Japan,

**Claims**

1. A method for assaying cholesterol in a low density lipoprotein in biological specimens comprising:

   a step of adding calixarenes to the specimen to form complexes of the calixarene and the low density lipoprotein thereby inhibiting the release of cholesterol in the low density lipoproteinso that the enzyme reaction of cholesterol in the low density lipoprotein, is inhibited;
   in a preliminary reaction an eliminating step of eliminating cholesterol in lipoproteins other than the low density lipoprotein, and
   in a second reaction a step of measuring the remaining cholesterol in the low density lipoprotein which has been inhibited from releasing cholesterol in the preliminary reaction.

2. A method for assaying cholesterol in a high density lipoprotein, in biological specimens comprising:

a step of adding calixarenes to the specimen to form complexes of the calixarenes and lipoproteins other than the high density lipoprotein thereby inhibiting the release of cholesterol in the lipoproteins other than the high density lipoprotein so that the enzyme reaction of cholesterol is inhibited in the lipoproteins other than the high density lipoprotein; and
a step for adding an enzyme to the specimen after adding calixarenes to the specimens to measure cholesterol in the high density lipoprotein.

3. The method of claim 1 or 2, wherein the calixarenes are at least one or more member selected from the group consisting of calix(4)arene, calix(6)arene, calix(8)arene, calix(4)arene sulfate, calix(6)arene sulfate, calix(8)arene sulfate, calix(4)arene acetate, calix(6)arene acetate, calix(8)arene acetate, carboxycalix(4)arene, carboxycalix(6) arene, carboxycalix(8)arene, calix(4)areneamine, calix(6)areneamine, and calix(8)areneamine.

4. A reagent for assaying cholesterol in a low density lipoprotein in biological specimens comprising:

calixarenes forming complexes with the low density lipoprotein, wherein a final concentration of the calixarenes is below 0.7 mM; and
one or more kinds of enzymes selected from the group consisting of lipoprotein
lipase, cholesterol esterase, cholesterol dehydrogenase, and cholesterol oxidase.

5. A reagent for assaying cholesterol in a high density lipoprotein, in biological specimens comprising:

calixarenes forming complexes with lipoproteins other than the high density lipoprotein wherein a final concentration of the calixarenes is in the range of 0.7 to 5 mM; and
one or more kinds of enzymes selected from the group consisting of lipoprotein lipase, cholesterol esterase, cholesterol dehydrogenase, and cholesterol oxidase.

6. The reagent of claim 4 or 5, wherein the calixarenes are at least one or more member selected from the group consisting of calix(4)arene, calix(6)arene, calix(8)arene, calix(4)arene sulfate, calix(6)arene sulfate, calix(8)arene sulfate, calix(4)arene acetate, calix(6)arene acetate, calix(8)arene acetate, carboxycalix(4)arene, carboxycalix(6) arene, carboxycalix(8)arene, calix(4)areneamine, calix(6)areneamine, and calix(8)areneamine.

7. The reagent of any of claims 4 to 6, which in the case of cholesterol dehydrogenase further comprises hydrazines.

8. The reagent of any of claims 4 to 7, which in the case of cholesterol dehydrogenase further comprises a coenzyme selected from the group consisting of Beta-nicotinamide adenine dinucleotide oxidation form (NAD), Thio-nicotinamide adenine dinucleotide oxidation form (t-NAD), Beta-nicotinamide adenine dinucleotide phosphate oxidation form (NADP), and Thio-nicotinamide adenine dinucleotide phosphate oxidation form (t-NADP).

9. A use of a reagent in a method for assaying cholesterol in a low density lipoprotein in biological specimen according to claim 1, the reagent comprising calixarenes forming complexes with the low density lipoprotein and one or more kinds of enzymes selected from the group consisting of lipoprotein lipase, cholesterol esterase, cholesterol dehydrogenase, and cholesterol oxidase.

10. A use of a reagent in a method for assaying cholesterol in a high density lipoprotein in biological specimen according to claim 2, the reagent comprising calixarenes forming complexes with low density lipoprotein and very low density lipoprotein and one or more kinds of enzymes selected from the group consisting of lipoprotein lipase, cholesterol esterase, cholesterol dehydrogenase, and cholesterol oxidase.

**Patentansprüche**

1. Verfahren zum Bestimmen von Cholesterin in einem Lipoprotein mit niederer Dichte in biologischen Proben, umfassend:

einen Schritt des Zugebens von Calixarenen zu der Probe, um Komplexe von dem Calixaren und dem Lipoprotein mit niederer Dichte zu bilden, um dadurch die Freisetzung von Cholesterin in dem Lipoprotein mit niederer Dichte zu hemmen, so dass die Enzym-Reaktion von Cholesterin in dem Lipoprotein mit niederer Dichte gehemmt wird;

in einer vorangehenden Reaktion einen Entfernungs-Schritt zum Entfernen von Cholesterin in Lipoproteinen, die von dem Lipoprotein mit niederer Dichte verschieden sind, und

in einer zweiten Reaktion einen Schritt des Messens von dem verbleibenden Cholesterin in dem Lipoprotein mit niederer Dichte, welches am Freisetzen von Cholesterin in der vorangehenden Reaktion gehemmt wurde.

2. Verfahren zum Bestimmen von Cholesterin in einem Lipoprotein mit hoher Dichte in biologischen Proben, umfassend:

einen Schritt des Zugebens von Calixarenen zu der Probe, um Komplexe von den Calixarenen und Lipoproteinen, die von dem Lipoprotein mit hoher Dichte verschieden sind, zu bilden, um dadurch die Freisetzung von Cholesterin in den Lipoproteinen, die von dem Lipoprotein mit hoher Dichte verschieden sind, zu hemmen, so dass die Enzym-Reaktion von Cholesterin in den Lipoproteinen, die von dem Lipoprotein mit hoher Dichte verschieden sind, gehemmt wird; und

einen Schritt des Zugebens von einem Enzym zu der Probe nach dem Zugeben von Calixarenen zu den Proben, um Cholesterin in dem Lipoprotein mit hoher Dichte zu
messen.

3. Verfahren nach Anspruch 1 oder 2, wobei die Calixarene mindestens eines oder mehrere Mitglieder, ausgewählt aus der Gruppe, bestehend aus Calix(4)aren, Calix(6)aren, Calix(8)aren, Calix(4)aren-sulfat, Calix(6)aren-sulfat, Calix(8)aren-sulfat, Calix(4)-aren-acetat, Calix(6)aren-acetat, Calix(8)aren-acetat, Carboxycalix(4)aren, Carboxycalix(6)aren, Carboxycalix(8)aren, Calix(4)aren-amin, Calix(6)aren-amin und Calix(8)aren-amin, darstellen.

4. Reagenz zum Bestimmen von Cholesterin in einem Lipoprotein mit niederer Dichte in biologischen Proben, umfassend:

Calixarene, die mit dem Lipoprotein mit niederer Dichte Komplexe bilden, wobei eine End-Konzentration von den Calixarenen unter 0,7 mM liegt; und

eine oder mehrere Arten von Enzymen, ausgewählt aus der Gruppe, bestehend aus Lipoprotein-Lipase, Cholesterin-Esterase, Cholesterin-Dehydrogenase und Cholesterin-Oxidase.

5. Reagenz zum Bestimmen von Cholesterin in einem Lipoprotein mit hoher Dichte in biologischen Proben, umfassend:

Calixarene, die mit Lipoproteinen, die von dem Lipoprotein mit hoher Dichte verschieden sind, Komplexe bilden, wobei eine End-Konzentration von den Calixarenen in dem Bereich von 0,7 bis 5 mM liegt; und

eine oder mehrere Arten von Enzymen, ausgewählt aus der Gruppe, bestehend aus Lipoprotein-Lipase, Cholesterin-Esterase, Cholesterin-Dehydrogenase und Cholesterin-Oxidase.

6. Reagenz nach Anspruch 4 oder 5, wobei die Calixarene mindestens eines oder mehrere Mitglieder, ausgewählt aus der Gruppe, bestehend aus Calix(4)aren, Calix(6)aren, Calix(8)aren, Calix(4)aren-sulfat, Calix(6)aren-sulfat, Calix(8)aren-sulfat, Calix(4)-aren-acetat, Calix(6)aren-acetat, Calix(8)aren-acetat, Carboxycalix(4)aren, Carboxycalix(6)aren, Carboxycalix(8)aren, Calix(4)aren-amin, Calix(6)aren-amin und Calix(8)aren-amin, darstellen.

7. Reagenz nach einem der Ansprüche 4 bis 6, das in dem Fall von Cholesterin-Dehydrogenase weiterhin Hydrazine umfasst.

8. Reagenz nach einem der Ansprüche 4 bis 7, das in dem Fall von Cholesterin-Dehydrogenase weiterhin ein Coenzym, ausgewählt aus der Gruppe, bestehend aus β-Nicotinamid-adenin-dinucleotid-oxidationsform (NAD), Thio-nicotinamid-adenindinucleotid-oxidationsform (t-NAD), ß-Nicotinamid-adenin-dinucleotid-phosphatoxidationsform (NADP) und Thio-nicotinamid-adenin-dinucleotid-phosphat-oxidationsform (t-NADP), umfasst.

9. Verwendung von einem Reagenz in einem Verfahren zum Bestimmen von Cholesterin in einem Lipoprotein mit niederer Dichte in einer biologischen Probe nach Anspruch 1, wobei das Reagenz Calixarene, die mit dem Lipoprotein mit niederer Dichte Komplexe bilden, und eine oder mehrere Arten von Enzymen, ausgewählt aus der Gruppe, bestehend aus Lipoprotein-Lipase, Cholesterin-Esterase, Cholesterin-Dehydrogenase und Cholesterin-Oxidase, umfasst.

10. Verwendung von einem Reagenz in einem Verfahren zum Bestimmen von Cholesterin in einem Lipoprotein mit hoher Dichte in einer biologischen Probe nach Anspruch 2, wobei das Reagenz Calixarene, die mit Lipoprotein mit niederer Dichte und Lipoprotein mit sehr niederer Dichte Komplexe bilden, und eine oder mehrere Arten von Enzymen, ausgewählt aus der Gruppe, bestehend aus Lipoprotein-Lipase, Cholesterin-Esterase, Cholesterin-Dehydrogenase und Cholesterin-Oxidase, umfasst.

## Revendications

1. Procédé de dosage de cholestérol dans une lipoprotéine de basse densité dans des spécimens biologiques, comprenant :

   une étape d'addition de calixarènes au spécimen pour former des complexes des calixarènes et de la lipoprotéine de basse densité, inhibant ainsi la libération de cholestérol dans la lipoprotéine de basse densité, de sorte que la réaction enzymatique du cholestérol dans la lipoprotéine de basse densité est inhibée ;
   lors d'une réaction préliminaire, une étape d'élimination pour éliminer du cholestérol dans des lipoprotéines autres que la lipoprotéine de basse densité, et
   lors d'une seconde étape de réaction, une étape de mesure du cholestérol restant dans la lipoprotéine de basse densité dont la libération de cholestérol a été inhibée pendant la réaction préliminaire.

2. Procédé de dosage de cholestérol dans une lipoprotéine de haute densité dans des spécimens biologiques, comprenant :

   une étape d'addition de calixarènes au spécimen pour former des complexes des calixarènes et de lipoprotéines autres que la lipoprotéine de haute densité, inhibant ainsi la libération de cholestérol dans les lipoprotéines autres que la lipoprotéine de haute densité, de sorte que la réaction enzymatique du cholestérol est inhibée dans les lipoprotéines autres que la lipoprotéine de haute densité ; et
   une étape d'addition d'une enzyme au spécimen après l'addition de calixarènes aux spécimens pour mesurer le cholestérol dans la lipoprotéine de haute densité.

3. Procédé selon la revendication 1 ou 2, dans lequel les calixarènes sont au moins un ou plusieurs éléments choisis dans l'ensemble constitué des calix(4)arène, calix(6)arène, calix(8)arène, sulfate de calix(4)arène, sulfate de calix(6)arène, sulfate de calix(8)arène, acétate de calix(4)arène, acétate de calix(6)arène, acétate de calix(8)arène, carboxycalix (4) arène, carboxycalix(6)arène, carboxycalix(8)arène, calix(4)arèneamine, calix(6)arèneamine et calix(8)arèneamine.

4. Réactif pour le dosage de cholestérol dans une lipoprotéine de basse densité dans des spécimens biologiques, comprenant :

   des calixarènes formant des complexes avec la lipoprotéine de basse densité, dans lesquels la concentration finale des calixarènes est inférieure à 0,7 mM ; et
   un ou plusieurs types d'enzymes choisies dans l'ensemble constitué des lipoprotéine lipase, cholestérol estérase, cholestérol déshydrogénase et cholestérol oxydase.

5. Réactif pour le dosage de cholestérol dans une lipoprotéine de haute densité dans des spécimens biologiques, comprenant :

   des calixarènes formant des complexes avec des lipoprotéines autres que la lipoprotéine de haute densité, dans lesquels la concentration finale des calixarènes est comprise dans la plage de 0,7 à 5 mM ; et
   un ou plusieurs types d'enzymes choisies dans l'ensemble constitué des lipoprotéine lipase, cholestérol estérase, cholestérol déshydrogénase et cholestérol oxydase.

6. Réactif selon la revendication 4 ou 5, dans lequel les calixarènes sont au moins un ou plusieurs éléments choisis dans l'ensemble constitué des calix(4)arène, calix(6)arène, calix(8)arène, sulfate de calix(4)arène, sulfate de calix(6)arène, sulfate de calix(8)arène, acétate de calix(4)arène, acétate de calix(6)arène, acétate de calix(8)arène, carboxycalix(4)arène, carboxycalix (6) arène, carboxycalix(8)arène, calix(4)arèneamine, calix(6)arèneamine et calix(8)arèneamine.

7. Réactif selon l'une quelconque des revendications 4 à 6, qui, dans le cas de la cholestérol déshydrogénase, comprend en outre des hydrazines.

8. Réactif selon l'une quelconque des revendications 4 à 7, qui, dans le cas de la cholestérol déshydrogénase, comprend en outre une coenzyme choisie dans l'ensemble constitué de la forme d'oxydation du bêta-nicotinamide adénine dinucléotide (t-NAD), la forme d'oxydation du bêta-nicotinamide adénine dinucléotide phosphate (NADP) et de la forme d'oxydation du thio-nicotinamide adénine dinucléotide phosphate (t-NADP).

9. Utilisation d'un réactif dans un procédé de dosage de cholestérol dans une lipoprotéine de basse densité dans un spécimen biologique selon la revendication 1, le réactif comprenant des calixarènes formant des complexes avec la lipoprotéine de basse densité et un ou plusieurs types d'enzymes choisies dans l'ensemble constitué des lipoprotéines lipase, cholestérol estérase, cholestérol déshydrogénase et cholestérol oxydase.

10. Utilisation d'un réactif dans un procédé de dosage de cholestérol dans une lipoprotéine de haute densité dans un spécimen biologique selon la revendication 2, le réactif comprenant des calixarènes formant des complexes avec une lipoprotéine de basse densité et une lipoprotéine de très basse densité et un ou plusieurs types d'enzymes choisies dans l'ensemble constitué des lipoprotéines lipase, cholestérol estérase, cholestérol déshydrogénase et cholestérol oxydase.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2600065 B
- JP HEI8201393 B
- JP 8201393 A
- JP HEI831195 B
- JP 8031195 A
- JP HEI996637 B
- JP 9096637 A
- JP HEI6242110 B
- JP 6242110 A
- JP 8131197 A
- JP HEI5176797 B
- JP HEI9169281 B

**Non-patent literature cited in the description**

- **DOMYAKU KOKA.** Standard Level for Total Cholesterol and Basis for Its Setting. *Arteriosclerosis,* 1966, vol. 24 (6), 260
- **FRIEDEWALD WT et al.** *Clin. Chem.,* 1972, vol. 18, 499-502
- **WARNICK GR et al.** *Clin. Chem.,* 1990, vol. 36 (1), 15-19
- **MCNAMARA, JR et al.** *Clin. Chem.,* 1990, vol. 36 (1), 36-42
- **ROGERS, R.D. et al.** *J. Radioanal. Nucl. Chem.,* 1996, vol. 208, 153-161